Europäisches Patentamt

European Patent Office

Office européen des brevets

(19)

(11)    EP 0 670 366 B1

(12)    **FASCICULE DE BREVET EUROPEEN**

(45) Date de publication et mention
de la délivrance du brevet:
**06.06.2001  Bulletin 2001/23**

(51) Int Cl.[7]: **C12N 9/04**, C12N 9/08,
A61K 35/78, A61K 7/48

(21) Numéro de dépôt: 95400449.5

(22) Date de dépôt: 02.03.1995

(54) **Extrait protéique de Cucumis melo à activité antioxydante et procédé de préparation, composition cosmétique ou pharmaceutique ou composition alimentaire contenant un tel extrait**

Proteinextrakt aus Cucumis melo mit Antioxydationswirkung und Verfahren zur Herstellung, kosmetische oder pharmazeutische Zubereitung oder Nahrungszubereitung die so ein Extrakt enthält

Proteic extract from Cucumis melo with antioxidant activity and process of preparation, cosmetic or pharmaceutic composition or food composition containing such extract

(84) Etats contractants désignés:
**AT BE CH DE DK ES FR GB GR IE IT LI LU MC NL PT SE**

(30) Priorité:  **03.03.1994  FR 9402459**

(43) Date de publication de la demande:
**06.09.1995  Bulletin 1995/36**

(73) Titulaire: **BIO-OBTENTION SC**
**F-34980 Motferrier-sur-Lez (FR)**

(72) Inventeurs:
• **Ginoux, Jean-Paul**
**F-13630 Eyragues (FR)**
• **Dreyer, Alain**
**F-84470 Chateauneuf de Gadagne (FR)**
• **Roch, Philippe**
**F-13630 Eyragues (FR)**

• **Baccou , Jean - Claude**
**F - 34080 Montpellier (FR)**
• **Lacan , Dominique**
**F - 34190 St Beauzille de Putois (FR)**

(74) Mandataire: **Warcoin, Jacques**
**Cabinet Régimbeau**
**20, rue de Chazelles**
**75847 Paris cedex 17 (FR)**

(56) Documents cités:
**FR-A- 1 404 428**

• **DATABASE WPI Section Ch, Week 8921, Derwent Publications Ltd., London, GB; Class D21, AN 89-153904 & JP-A-1 093 508 (SUGIURA SHINYAKU) 12 Avril 1989**

EP 0 670 366 B1

**Description**

La présente invention a pour objet un extrait protéique de Cucumis melo présentant une activité enzymatique améliorée superoxyde dismutase et éventuellement catalase et un procédé de préparation dudit extrait protéique.

Elle a également pour objet une composition pharmaceutique utilisable dans le traitement des cancers ou une composition pharmaceutique ou cosmétique pour usage topique externe, notamment contre le vieillissement de la peau, comportant un tel extrait. Elle a également pour objet une composition agroalimentaire contenant un tel extrait.

On sait depuis longtemps que l'oxygène atmosphérique est un des agents responsables de la détérioration de la matière organique exposée à l'air. De ce fait, la recherche de substances anti-oxydantes a fait l'objet d'un intérêt constant aussi bien dans le domaine de la cosmétologie, de l'agroalimentaire ou de la médecine.

Le brevet FR-B-2 287 899 a décrit par exemple l'application en cosmétologie des enzymes superoxyde dismutases et en particulier l'utilisation de ces enzymes dans la préparation de compositions cosmétiques pour le soin de la peau et des cheveux.

Les superoxyde-dismutases sont des enzymes capables d'induire la dismutation des ions superoxyde, suivant la réaction :

$$2\ O_2 + 2H+ \rightarrow H_2O_2 + O_2$$

Ces superoxyde-dismustases sont notamment extraites d'érythrocytes de boeuf (Markovitz, J. Biol. Chem. 234, p. 40, 1959), d'Escherichia coli (Keele et Fridovitch, J. Biol., 245, p. 6176, 1970) et de souches bactériennes marines (brevets FR 2 225 443 et 2 240 277).

Néanmoins, pour des raisons liées notamment aux substances infectieuses que peuvent contenir ces matières, on a tendance à l'heure actuelle à remplacer celles-ci par des substances provenant du règne végétal réputées plus saines. Ces enzymes anti-oxydantes sont en effet présentes dans des quantités variables dans certains végétaux. Il est également connu que les superoxyde-dismutases sont susceptibles d'avoir des actions anti-cancéreuses au niveau du système digestif par piégeage des radicaux libres $O_2-$ (communication du Professeur Crastes de Paulet de la Faculté de Médecine et de l'INSERM de Montpellier).

L'action des catalases sur l'eau oxygénée résultant de l'action des superoxyde-dismutases permettrait d'augmenter considérablement les effet de ces dernières par élimination de l'eau oxygénée produite.

De nos jours, les antioxydants de synthèse pour la conservation des aliments sont couramment utilisés. Les deux principaux antioxydants de synthèse utilisés sont le butylhydroxytoluène (BHT) ou le butylhydroxyanizole (BHA). Cependant, on a montré récemment que ces molécules de synthèse pouvaient être toxiques, notamment cancérigènes, et donc dangereuses à certaines doses, d'où une recherche de substances anti-oxydantes d'origine végétale pour les remplacer. Hemeda et al, 1990, J. of Food Science 55, 1, 184 et Farag et al, 1989, JAOCS, 66, 6, p. 800) ont proposé de remplacer ces composés par des substances issues de végétaux.

Dans le domaine des fruits, on a noté chez certains fruits climatériques comme le melon (Cucumis melo) des activités superoxyde dismutases de l'ordre de 22 à 24 unités enzymatiques par mg de protéines et une activité catalase de l'ordre de 35 à 40 unités enzymatiques par mg de protéines. Dans le cas de la tomate, l'activité superoxyde dismutase se situe aux alentours de 10 unités d'activités enzymatiques par mg de protéines.

Les fruits climatériques se distinguent des fruits non climatériques, notamment par le mode de maturation. La phase de maturation des fruits correspond à un phénomène oxydatif à partir duquel on observe une destruction des systèmes membranaires et un ralentissement de l'activité métabolique. Concrètement, les fruits dits climatériques dégagent de l'éthylène de façon autocatalytique au début de la phase de maturation. Chez ces fruits, l'éthylène semble initier, coordonner et accélérer l'ensemble des processus physiologiques de maturation.

Par contre, les fruits non climatériques ne présentent pas cette crise éthylénique, autocatalytique au début de leur phase de mûrissement, et il n'est pas possible de modifier la maturation par application d'éthylène.

Les fruits non climatériques sont notamment les agrumes, les raisins et les fraises et les fruits climatériques sont notamment les pommes, les poires, les tomates, les melons et les bananes.

De façon inattendue, on a trouvé de nouveaux extraits protéiques de Cucumis melo qui présentaient une activité enzymatique superoxyde dismutase et éventuellement catalase améliorée.

On a également trouvé que les nouveaux extraits pouvaient être avantageusement utilisés en cosmétologie, notamment contre le vieillissement de la peau, sous la forme d'une composition cosmétique appropriée.

Un autre objet de la présente invention est de proposer une nouvelle composition pharmaceutique contenant à titre de principe actif ces nouveaux extraits protéiques utiles notamment dans le traitement de certains cancers.

Un autre objet de la présente invention est de proposer une composition alimentaire contenant de tels extraits protéiques à titre d'antioxydants en remplacement notamment des molécules de synthèse antioxydantes BHT et BHA ou analogue.

De ce fait, l'invention concerne en premier lieu un extrait protéique, caractérisé en ce qu'il provient de Cucumis melo et qu'il présente une activité enzymatique superoxyde dismutase supérieure à 30 unités enzymatiques par mg de protéines solubles ainsi que de préférence une activité enzymatique catalyse supérieure à 45 unités enzymatiques par mg de protéines solubles.

De façon générale, les extraits protéiques selon l'invention présentent une activité superoxyde dismutase et éventuellemnt catalase améliorée par rapport aux extraits protéiques connus obtenus à partir de la plupart des melons. Les extraits selon l'invention résultent d'une sélection parmi un grand nombre de melons.

Par unité (U) d'activité catalase, on désigne le nombre de millimole de $H_2O_2$ réduite par minute. De façon connue, 1 gramme de matière fraiche d'un melon fournit environ 10 mg de protéines solubles.

L'activité superoxyde-dismutase est mesurée de façon connue en unité par mg de protéines solubles. On doit comprendre que dans sa forme la plus générale, l'extrait protéique selon l'invention est tel que pour 1 gramme de matière fraîche, on recueille 10 mg de protéines solubles.

Dans les mêmes conditions, un extrait protéique d'un melon quelconque conduira à une activité SOD et catalase très inférieure (de deux à quatre fois ou plus selon les cas).

L'extrait peut donc comprendre également d'autres protéines solubles de Cucumis melo selon le procédé d'extraction utilisé.

Selon une variante préférée, l'extrait protéique selon l'invention présente une activité enzymatique superoxyde dismutase supérieure à 50 unités enzymatiques par mg et éventuellement une activité enzymatique catalase supérieure à 60 unités enzymatiques par mg, avantageusement une activité enzymatique superoxyde dismustase supérieure à 60 unités par mg et éventuellement une activité enzymatique catalase supérieure à 100 unités par mg.

De préférence encore, l'activité SOD est supérieure à 80 unités par mg et éventuellement l'activité catalase supérieure à 100 unités par mg ou éventuellement 110 unités par mg.

Cet extrait contient donc de façon inattendue une ou plusieurs enzymes possédant une activité SOD et une ou plusieurs enzymes possédant une activité catalase selon les valeurs indiquées précédemment.

Selon une autre variante préférée, l'extrait protéique provient d'un Cucumis melo présentant une production d'éthylène stable après la crise éthylénique, c'est-à-dire que la production d'éthylène présente un plateau qui peut être de plusieurs jours. Dans le cas général, les fruits climatériques comme Cucumis melo présentent la propriété, sur le plan de la conservation, de passer très rapidement de l'état mature à l'état surmature après l'apparition du paroxysme de la crise éthylénique. Comme cela est bien connu, la crise éthylénique chez Cucumis melo est très importante et peu de temps après, le fruit commence à se désorganiser et sa valeur marchande diminue. Au contraire, dans le cas des Cucumis melo qui sont susceptibles de fournir un extrait protéique selon l'invention, l'émission d'éthylène présente un plateau stable de préférence pendant au moins 5 jours après la crise éthylénique et encore plus avantageusement pendant au moins 7 jours.

Ce type de Cucumis melo est notamment décrit dans la demande de brevet internationale WO 92/02622 dont le titulaire est la société déposante.

Selon cette demande de brevet, la variété de Cucumis melo obtenue par croisement génétique présente une durée de conservation supérieure (de l'ordre de 10 à 14 jours ou plus) à celle des variétés traditionnelles qui est de 5 jours environ.

Une étude macroscopique réalisée sur des tranches de Cucumis melo tel que décrit dans WO 92/02622 montre que les structures tissulaires ne sont pas altérées par la maturation (on n'observe ni vitrescence, ni liquéfaction liées à la destruction des cellules et caractéristiques des tissus sénescents chez le Cucumis melo).

Sans que le déposant ne veuille être lié par une quelconque interprétation scientifique, on peut émettre l'hypothèse que la durée de conservation importante du Cucumis melo tel qu'il est décrit dans la demande internationale précitée pourrait être liée à la présence de quantité importante de substances anti-oxydantes.

L'homme du métier au vu de cette demande de brevet disposera aisément des Cucumis melo susceptibles de produire un extrait protéique selon l'invention.

En particulier, à partir de la lignée 95LS444 de Cucumis melo dont les semences ont été déposées conformément au Traité de Budapest dans la collection NCIMB (National Collection of Industrial and Marine Bacteria-ABERDEEN AB2 IRY (Ecosse - GB) 23 St. Machar Drive) le 19 Juillet 1990 sous le numéro 40310, on peut par hybridation obtenir d'autres variétés de Cucumis melo par exemple des variétés du type Vauclusien, Clipper et Supporter présentant les mêmes caractéristiques permettant de conduire aux extraits protéiques selon l'invention.

L'invention a donc également pour objet un extrait protéique soluble tel qu'il peut être obtenu à partir d'un Cucumis melo présentant un plateau de production d'éthylène après la crise éthylénique, avantageusement pendant au moins cinq jours, de préférence pendant au moins sept jours.

De préférence, l'extrait protéique est tel qu'il peut être obtenu à partir de la lignée cellulaire 95LS444 ou de l'une des lignées hybrides issues de 95LS444.

Ces extraits protéiques sont tels qu'ils peuvent être obtenus par un procédé quelconque dans l'art permettant de récupérer la substance soluble.

Avantageusement, ces extraits protéiques sont tels qu'ils puissent être obtenus par broyage ou pressage en milieu aqueux d'un Cucumis melo tel que décrit précédemment à pH d'environ 7,5 suivi de la récupération du surnageant.

L'invention a également pour objet un procédé de préparation d'extraits protéiques de Cucumis melo tels que décrits précédemment, caractérisé en ce qu'on broie en milieu aqueux à un pH approprié des Cucumis melo qui présentent un plateau de production d'éthylène après le paroxysme éthylénique tels que décrits précédemment et que l'on récupère notamment par centrifugation ou filtration le surnageant pour purification éventuelle ultérieure.

Le pH pour un tel procédé est de 5 9 de préférence, ce qui permet de rester dans des conditions physicologiques optimales (sans dénaturation des SOD et catalases).

De préférence, il s'agit d'une centrifugation permettant d'évacuer les débris membranaires.

Ces cucumis melo sont de préférence ceux décrits dans la demande de brevet WO 92/02622.

La présente invention a également pour objet une composition pharmaceutique ou cosmétique pour usage topique externe, notamment contre le vieillissement de la peau ou pour le soin des cheveux, contenant à titre de principe actif un extrait protéique de Cucumis melo présentant une activité enzymatique superoxyde dismutase supérieure à 30 unités enzymatiques par mg et avantageusement une activité enzymatique catalase supérieure à 45 unités enzymatiques par mg.

La présente invention concerne également une composition cosmétique ou pharmaceutique contenant les extraits protéiques des variantes préférées telles que définies précédemment.

Cette composition cosmétique présente en plus de l'extrait protéique un véhicule inerte tel qu'il est bien connu dans l'art de la cosmétologie.

De préférence, l'extrait protéique sera en quantité suffisante de façon à ce que la composition cosmétique appliquée sur la peau procure un effet positif contre le vieillissement ou procure un effet positif sur les cheveux.

En général, la quantité d'extrait protéique est en quantité telle que la composition cosmétique comprend 0,01 à 5 % en poids de superoxyde dismutase, de préférence 0,01 à 1 %.

Ces compositions sont notamment décrites dans le brevet FR-A-2 287 899 auquel l'homme du métier pourra se référer et sont par exemple des solutions du type lotions, des émulsions de consistance liquide ou semi-liquide de type lait, obtenues par dispersion d'une phase grasse dans une phase aqueuse ou inversement ou de suspensions ou émulsions de consistance molle du type crème ou gel. Ces compositions sont préparées selon les méthodes usuelles.

Parmi les véhicules inertes utilisés de façon usuelle dans les formulations pour la peau mentionnées ci-dessus, on peut citer les tensioactifs, les colorants, les parfums, les agents conservateurs, les émulsionnants, les véhicules liquides tels que l'eau, des corps gras tels que les huiles naturelles ou synthétiques destinées à constituer la phase grasse des laits ou des crèmes, des résines du type carboxylvinylique ou anhydride maléique neutralisées par des amines tertiaires et notamment par des aminoalcools tels que la triéthanolamine.

Les compositions pour cheveux selon l'invention peuvent être sous forme de solutions aqueuses, alcooliques ou hydroalcooliques, ou sous forme de crèmes, de gels, d'émulsions ou encore sous forme de bombes aérosols contenant également un agent propulseur sous pression. Elles constituent par exemple des shampooings, des lotions de mise en plis, des lotions traitantes, des crèmes ou des gels coiffants, des compositions de teintures etc. Ces compositions peuvent renfermer divers adjuvants habituellement présents dans ces compositions pour cheveux, par exemple des parfums, des colorants, des agents conservateurs, des agents séquestrants, des agents épaississants etc.

L'invention a également pour objet une composition pharmaceutique utile notamment dans le traitement de certains cancers digestifs contenant à titre de principe actif un extrait protéique de Cucumis melo tel que défini précédemment, en association avec un ou plusieurs véhicules inertes adaptés à cette utilisation.

Ces compositions peuvent être administrées sous forme orale, parentérale, ou par toute autre voie compatible avec le traitement recherché.

L'invention a également pour objet une composition alimentaire contenant en tant qu'antioxydant un extrait protéique de Cucumis melo tel que défini précédemment à une dose efficace.

L'invention est maintenant illustrée par les exemples suivants donnés à titre indicatif sans qu'ils limitent de quelque manière la portée de l'invention.

Exemples 1 à 8:

Préparation des extraits et mesure des activités enzymatiques d'extraits de Cucumis melo selon l'invention, à différents stades de maturation

5 g de pulpe d'un Cucumis melo hybride issu de la lignée cellulaire 95LS444 (décrite dans la demande internationale WO 92/02622) sont utilisés à différents stades de maturation qui constituent les différents exemples 1 à 8.

La courbe de production d'éthylène de cette variété est indiquée à la figure unique annexée, à partir du premier jour de la récolte et a déjà été décrite dans la demande internationale précitée du déposant.

Selon un mode général de préparation : 5 g de pulpe sont broyés dans un mortier au froid. Un volume de tampon Phosphate 50 mM (pH : 7,5; 5EDTA 1 mM ; glycérol 5 %) équivalent à 3 fois la masse du végétal est ajouté.

Après homogénéisation, la suspension a été centrifugée à 5000 g à 4°C pendant 30 minutes. Le surnageant est ensuite récupéré et filtré, cet extrait brut sert à la détermination des activités catalases et superoxyde dismutases de même que des teneurs en protéines.

L'activité catalase est mesurée en suivant à 230 nm et à 25°C la disparition du substrat $H_2O_2$. L'activité superoxyde dismustase a été mesurée à l'aide d'un kit de dosage SOD 525 fourni par la société Bioxytech. Cette méthode donne des résultats comparables à ceux obtenus par la méthode de McCord et Fridovitch (inhibition par la SOD de la réduction du cytochrome C par la xanthine-xanthine oxydase). Les protéines solubles ont été mesurées selon la méthode de Bradford (1976).

Les résultats sont réunis dans le tableau I ci-dessous.

TABLEAU I

| Exemples | Jours après récolte | Activité SOD U/mg protéines | Activité catalase U/mg protéines |
|----------|---------------------|-----------------------------|----------------------------------|
| 1 | 1 | 126 | 119 |
| 2 | 2 | 95,4 | 125 |
| 3 | 3 | --- | --- |
| 4 | 5 | 79,4 | 129 |
| 5 | 6 | --- | --- |
| 6 | 8 | 70 | 132 |
| 7 | 11 | 63 | 130 |
| 8 | 15 | 40 | 160 |

Exemples comparatifs 9 à 16:

Préparation des extraits et mesure des activités enzymatiques d'extraits de Cucumis melo témoin à différents stades de maturation

Les mêmes essais effectués sur des melons communs du type charentais aux mêmes phases de maturation que les Cucumis melo hybrides issus de la lignée 95LS444 sont reproduits.

Les résultats sont également indiqués dans le tableau II ci-dessous.

Ces résultats montrent que les activités catalases et superoxyde dismustases sont très importantes dans le cas des extraits protéiques selon l'invention.

TABLEAU II

| Exemples | Jours après récolte | Activité SOD U/mg protéines | Activité catalase U/mg protéines |
|----------|---------------------|-----------------------------|----------------------------------|
| 9 | 1 | 22,5 | 36,7 |
| 10 | 2 | 22,1 | 38,7 |
| 11 | 3 | 22,7 | 34,5 |
| 12 | 5 | 24 | 35,6 |
| 13 | 6 | 22,1 | 36 |
| 14 | 8 | --- | --- |
| 15 | 11 | --- | --- |
| 16 | 15 | --- | --- |

**Revendications**

1. Extrait protéique soluble, caractérisé en ce qu'il provient de Cucumis melo et qu'il présente une activité enzymatique superoxyde dismutase supérieure à 30 U/mg de protéines.

2. Extrait protéique soluble selon la revendication 1, caractérisé en ce qu'il présente une activité enzymatique catalase supérieure à 45 U/mg de protéines.

3. Extrait protéique soluble selon l'une des revendications 1 ou 2, caractérisé en ce qu'il présente une activité enzymatique superoxyde dismutase supérieure à 50 U/mg et éventuellement une activité enzymatique catalase supérieure à 60 U/mg.

4. Extrait protéique soluble selon l'une des revendications 1 ou 2, caractérisé en ce qu'il présente une activité enzymatique superoxyde dismutase supérieure à 60 U/mg et éventuellement une activité enzymatique catalase supérieure à 100 U/mg.

5. Extrait protéique soluble selon la revendication 4, caractérisé en ce qu'il présente une activité enzymatique superoxyde dismutase supérieure à 80 U/mg et éventuellement une activité enzymatique catalase supérieure à 100 U/mg.

6. Extrait protéique soluble selon la revendication 5, tel qu'il peut être obtenu à partir d'un Cucumis melo présentant un plateau de production d'éthylène après la crise éthylénique pendant au moins cinq jours, de préférence pendant au moins sept jours.

7. Extrait protéique soluble selon la revendication 5 ou 6, tel qu'il peut être obtenu à partir de la lignée cellulaire 95LS444 ou de l'une des lignées hybrides issues de 95LS444 et plus particulièrement des variétés commerciales type Vauclusien Clipper et Supporter.

8. Extrait protéique soluble selon la revendication 7, tel qu'il peut être obtenu par broyage ou pressage en milieu aqueux d'un Cucumis melo à pH de 5 à 9 puis récupération du surnageant.

9. Procédé de préparation d'extrait protéique soluble de Cucumis melo selon l'une des revendications 1 à 8, caractérisé en ce qu'on broie un Cucumis melo tel que défini aux revendications 6 à 8 et que l'on récupère par séparation le surnageant.

10. Procédé de préparation selon la revendication 9, caractérisé en ce que la séparation est effectuée par centrifugation ou tout autre technique de filtration.

11. Composition pharmaceutique ou cosmétique pour usage topique externe, contenant à titre de principe actif un extrait protéique éventuellement purifié selon l'une des revendications 1 à 8.

12. Composition selon la revendication 11, caractérisée en ce que la quantité d'extrait protéique est telle que la composition cosmétique comprend 0,01 à 5 % en poids de superoxyde dismutases, de préférence de 0,01 à 1 %.

13. Composition pharmaceutique notamment utile dans le traitement des cancers, contenant à titre de principe actif un extrait protéique éventuellement purifié selon l'une des revendications 1 à 8.

14. Composition alimentaire contenant à titre d'antioxydant un extrait protéique éventuellement purifié selon l'une des revendications 1 à 8.


**Patentansprüche**

1. Löslicher Proteinextrakt, dadurch gekennzeichnet, dass er von Cucumis melo stammt und eine enzymatische Superoxiddismutaseaktivität von über 30 U/mg Protein aufweist.

2. Löslicher Proteinextrakt gemäß Anspruch 1, dadurch gekennzeichnet, dass er eine enzymatische Katalaseaktivität von übor 45 U/mg Protein aufweist.

3. Löslicher Proteinextrakt gemäß einem der Ansprüche 1 oder 2, dadurch gekennzeichnet, dass er eine enzymatische Superoxiddismutaseaktivität von über 50 U/mg und gegebenenfalls eine enzymatische Katalaseaktivität von über 60 U/mg aufweist.

4. Löslicher Proteinextrakt gemäß einem der Ansprüche 1 oder 2, dadurch gekennzeichnet, dass er eine enzymatische Supeoroxiddismutaseaktivität von über 60 U/mg und gegebenenfalls eine enzymatische Katalaseaktivität von über 100 U/mg aufweist.

5. Löslicher Proteinextrakt gemäß Anspruch 4, dadurch gekennzeichnet, dass er eine enzymatische Superoxiddismutaseaktivität von über 80 U/mg und gegebenenfalls eine enzymatische Katalaseaktivität von über 100 U/mg aufweist.

6. Löslicher Proteinextrakt gemäß Anspruch 5, welcher ausgehend von einer Cucumis melo, welche ein Ethylenproduktionsplateau nach der ethylenischen Krise von wenigstens fünf Tagen, bevorzugt wenigstens sieben Tagen, aufweist, erhalten werden kann.

7. Löslicher Proteinextrakt gemäß Anspruch 5 oder 6, welcher ausgehend von der Zelllinie 95LS444 oder einer der von 95LS444 stammenden Hybridlinien, und insbesondere der Handelssorten des Typs Vauclusien Clipper und Supporter, erhalten werden kann.

8. Löslicher Proteinextrakt gemäß Anspruch 7, welcher durch Zerkleinern oder Pressen im wässrigen Milieu einer Cucumis melo bei pH 5 bis 9 und anschließende Gewinnung des Überstands erhalten werden kann.

9. Verfahren zur Herstellung eines löslichen Proteinextrakts von Cucumis melo gemäß einem der Ansprüche 1 bis 8, dadurch gekennzeichnet, dass man eine Cucumis melo, wie in den Ansprüchen 6 bis 8 definiert, zerreibt und den Überstand durch Abtrennung gewinnt.

10. Herstellungsverfahren gemäß Anspruch 9, dadurch gekennzeichnet, dass die Abtrennung durch Zentrifugation oder eine andere Filtrationstechnik bewirkt wird.

11. Pharmazeutische oder kosmetische Zusammensetzung zur äußerlichen topischen Anwendung, welche als Wirkstoff einen gegebenenfalls gereinigten Proteinextrakt gemäß einem der Ansprüche 1 bis 8 enthält.

12. Zusammensetzung gemäß Anspruch 11, dadurch gekennzeichnet, dass die Menge an Proteinextrakt derart ist, dass die kosmetische Zusammensetzung 0,01 bis 5 Gew.-%, bevorzugt 0,01 bis 1 %, Superoxiddismutase enthält.

13. Pharmazeutische Zusammensetzung, insbesondere zur Behandlung von Krebs geeignet, welche als Wirkstoff einen gegebenenfalls gereinigten Proteinoxtrakt gemäß einem der Ansprüche 1 bis 8 enthält.

14. Nahrungsergänzungsmittel, welches als Antioxidans einen gegebenenfalls gereinigten Proteinextrakt gemäß einem der Ansprüche 1 bis 8 enthält.

**Claims**

1. Soluble protein extract, characterized in that it is obtained from Cucumis melo and in that it has a super-oxide dismutase enzyme activity greater than 30 U/mg of proteins.

2. Soluble protein extract according to Claim 1, characterized in that it has a catalase enzyme activity greater than 45 U/mg of proteins.

3. Soluble protein extract according to either of Claims 1 and 2, characterized in that it has a superoxide dismutase enzyme activity greater than 50 U/mg and possibly a catalase enzyme activity greater than 60 U/mg.

4. Soluble protein extract according to either of Claims 1 and 2, characterized in that it has a superoxide dismutase enzyme activity greater than 60 U/mg and possibly a catalase enzyme activity greater than 100 U/mg.

5. Soluble protein extract according to Claim 4, characterized in that it has a superoxide dismutase enzyme activity greater than 80 u/mg and possibly a catalase enzyme activity greater than 100 u/mg.

6.  Soluble protein extract according to Claim 5, as can be obtained from a Cucumis melo having an ethylene production plateau after the ethylene crisis for at least five days, preferably for at least seven days.

7.  Soluble protein extract according to Claim 5 or 6, as can be obtained from the 95LS444 cell line or from one of the hybrid lines obtained from 95LS444 and more particularly from the commercial varieties of the Vauclusien, Clipper and Supporter type.

8.  Soluble protein extract according to Claim 7, as can be obtained by grinding or pressing, in aqueous medium, a Cucumis melo at a pH of 5 to 9, followed by recovery of the supernatant.

9.  Process for preparing soluble Cucumis melo protein extract according to one of Claims 1 to 8, characterized in that a Cucumis melo as defined in Claims 6 to 8 is ground and in that the supernatant is recovered by separation.

10. Preparation process according to Claim 9, characterized in that the separation is carried out by centrifu-gation or any other filtration technique.

11. Pharmaceutical or cosmetic composition for external topical use, containing as active ingredient an optionally purified protein extract according to one of Claims 1 to 8.

12. Composition according to Claim 11, characterized in that the quantity of protein extract is such that the cosmetic composition comprises 0.01 to 5 % by weight of superoxide dismutase, preferably 0.01 to 1 %.

13. Pharmaceutical composition especially useful in the treatment of cancers, containing as active ingredient an optionally purified protein extract according to one of Claims 1 to 8.

14. Food composition containing, as antioxidant, an optionally purified protein extract according to one of Claims 1 to 8.